# EUROPEAN PATENT APPLICATION

(11) **EP 2 543 373 A1**
(43) Date of publication of application: **09.01.2013**
(21) Application number: 10846870.3
(22) Date of filing: 17.06.2010
(51) Int. Cl.: A61K 31/4422, A61K 31/4035, A61K 9/20, A61K 9/48, A61P 9/12

(54) **COMPOUND PHARMACEUTICAL COMPOSITION OF LEVOAMLODIPINE**

(30) Priority: 03.03.2010 CN 201010116867
(71) Applicant: Shihuida Pharmaceuticals Group (Jilin) Ltd, Shanghai 200439 (CN)
(72) Inventor: YANG, Yanling, Shanghai 200439 (CN); XUE, Chuanxiao, Shanghai 200439 (CN); ZHANG, Xitian, Shanghai 200439 (CN); XU, Wei, Shanghai 200439 (CN); ZHANG, Yan, Shanghai 200439 (CN)
(74) Representative: Török, Ferenc
(86) International application number: PCT/CN2010/074004
(87) International publication number: WO 2011/106951

(57) **Abstract**

A levamlodipine compound pharmaceutical composition is provided, which contains levamlodipine or a pharmaceutically acceptable salt thereof, and chlorthalidone. In the present invention, levamlodipine and chlorthalidone are administrated in combination for treating hypertension, a high synergistic antihypertensive effect is achieved, and the edema side effect due to sodium and water retention caused when levamlodipine is administrated alone is mitigated. In addition, levamlodipine increases insulin sensitivity of an organism, and resists the side effect of increase in blood glucose level caused by chlorthalidone. The pharmaceutical composition of levamlodipine and chlorthalidone overcomes the disadvantage that levamlodipine takes effects slowly in hypertension repression.

## Description

### BACKGROUND

### Technical Field

The present invention relates to the pharmaceutical field, and more particularly to a levamlodipine compound pharmaceutical composition.

### Related Art

Hypertension is a clinical syndrome characterized by elevation of systolic blood pressure (SBP) and/or diluted blood pressure (DBP) of arteries of systemic circulation, and is one of the mostly common cardiovascular diseases at present. According to the latest statistical data, hypertension incidence in China is up to 11.68% at present, and especially the secondary lesions of target organs such as heart, brain, and kidney of hypertension seriously influence longevity and quality of life of the patients.

Amlodipine is a third generation dihydropyridine calcium channel blocker and a long acting dihydropyridine hypertension depression drug, and has a chemical name 3-ethyl-5-methyl-2-(2-aminoethoxymethyl)-4-(2-chlorophenyl)-1, 4-dihydro-6-methyl-3,5-pyridine dicarboxylate. The compound has laevo and dextro isomorphic isomers, the calcium antagonism activity of the laevo isomer is 1000 times of that of the dextro isomer, and 2 times of that of a raceme, and the side effect is greatly lowered. Levamlodipine may be used to treat hypertension associated with heart failure, reverse ventricular hypertrophy, improve the relaxation function of the heart during the diastolic stage, protect the renal function with mild diuretic function, and prevent coronary heart disease, myocardial infarction, and stroke, and may further partially reverse abnormal circadian rhythm of blood pressure, and have mild anti-platelet effect, anti-myocardial ischemia effect, anti-arrhythmia effect, insulin sensitivity increasing effect and a certain anti-atherosclerosis effect. However, in the blood pressure depressing process, levamlodipine takes effect slowly, and sodium and water retention easily occurs due to the high dosage, thus resulting in side effects such as edema, so the drug is withdrawn for considerable patients, and the clinical application of levamlodipine is limited.

Chlorthalidone is a commonly used thiazide diuretic agent, and has a chemical name 5-(2,3-dihydro-1-hydroxy-3-oxo-1H-isoindol-1-yl)-2-chlorobenzenesulfonamide. In addition to depression of blood pressure by excretion of sodium through diuresis, and decrease of the blood volume to reduce the output from the heart, the hypertension depression mechanism thereof is mainly to diminish the response of arteriolar smooth muscle to pressure increasing substances by decreasing the sodium ion content in vascular sooth muscle, thereby diluting the vessel and lowering the blood pressure. Chlorthalidone has the advantage of fast acting hypertension depression effect; however, long-term use of chlorthalidone alone may cause water-electrolyte imbalance, hypokalemia, hyperglycemia due to inhibition of insulin release, and hyperuricemia due to the inference of excretion of uric acid by renal tubule.

### SUMMARY

In view of edema side effect of extremity end due to sodium and water retention caused by levamlodipine when administrated alone to depress blood pressure, and the side effects of hypokalemia, hyperglycemia, and hyperuricemia caused by chlorthalidone when administrated alone in the prior art, the present invention is directed to a levamlodipine compound pharmaceutical composition.

The levamlodipine compound pharmaceutical composition of the present invention includes levamlodipine or a pharmaceutically acceptable salt thereof and chlorthalidone.

In the present invention, levamlodipine and chlorthalidone are administrated in combination, so as to lower the dosages of levamlodipine and chlorthalidone in treatment, while the same or even a better blood pressure depression effect is achieved. Moreover, as levamlodipine and chlorthalidone are given at lower dosages, the side effects of edema due to sodium and water retention caused by levamlodipine, and hypokalemia, hyperglycemia, and hyperuricemia caused by chlorthalidone are lowered. In addition, levamlodipine can increase insulin sensitivity of an organism, and can resist hyperglycemia caused by chlorthalidone when administrated in combination with chlorthalidone. Besides, the edema side effect due to sodium and water retention caused by levamlodipine may be further decreased due to the diuretic property of indapamide.

In the present invention, the weight ratio of levamlodipine to chlorthalidone is 1:1-10, and preferably 1:5-7.5.

The pharmaceutically acceptable salt is one or more selected from acetate, benzenesulfonate, benzoate, citrate, fumarate, glucoheptonate, hydrochloride, lactate, maleate, malate, mesylate, nitrate, phosphate, succinate, phosphate, and tartrate.

The levamlodipine compound pharmaceutical composition of the present invention further includes a pharmaceutically acceptable adjuvant.

In the levamlodipine compound pharmaceutical composition of the present invention, the content of levamlodipine is preferably 0.8-4.0 wt%, and more preferably 2.0-4.0 wt%; and the content of chlorthalidone is preferably 4.0-40 wt%, and more preferably 10-20 wt%.

The pharmaceutically acceptable adjuvant is one or more selected from microcrystalline cellulose, pregelatinized starch, lactose, hydroxymethyl starch sodium, and magnesium stearate.

The levamlodipine compound pharmaceutical composition of the present invention further includes a pharmaceutically acceptable diluent, adhesive, disintegrant, lubricant, coloring agent and/or flavoring agent.

The levamlodipine compound pharmaceutical composition of the present invention may be prepared into an oral preparation, for example, a tablet or a capsule, in which the tablet may be sugar coated, film coated, or uncoated.

The present invention has the following effective and active effects. As levamlodipine and chlorthalidone are administrated in combination, a high synergistic antihypertensive effect is achieved. The dosages of levamlodipine and chlorthalidone are decreased, while the same or even better antihypertensive effect is achieved. Moreover, low dose of levamlodipine may also decrease the edema side effect due to sodium and water retention caused by levamlodipine, and the diuretic effect of chlorthalidone may further decrease the edema side effect due to sodium and water retention caused by levamlodipine. Furthermore, low dose of chlorthalidone may also decrease the side effects of hypokalemia, hyperglycemia, and hyperuricemia caused by chlorthalidone. In addition, levamlodipine can increase insulin sensitivity of an organism, and can resist hyperglycemia caused by chlorthalidone when administrated in combination with chlorthalidone. Due to the fast acting hypertension depression effect of chlorthalidone, the administration of levamlodipine and chlorthalidone in combination is useful in a patient at high risk of hypertension, thereby overcoming the characteristics of slow acting hypertension depression effect of levamlodipine. In addition, the administration of levamlodipine and chlorthalidone in combination has a significant effect for lowering the systolic pressure, thus being especially suitable for isolated systolic hypertension in elderly patients.

### BRIEF DESCRIPTION OF THE DRAWINGS

No drawings

### DETAILED DESCRIPTION

### Blood Pressure Repression Effect Embodiment 1

### 1.1 Experimental materials

Levamlodipine benzenesulfonate (commercially available, Shihuida®); chlorthalidone (purchased from Changzhou Xinli Medicines & Chemical Co., Ltd.); and other reagents, purchased from Sigma Corporation. Spontaneous hypertension (with a blood pressure higher than 200 mmHg) male Wistar rats (purchased from Laboratory Animal Medicine Department, Shanghai Medical College, Fudan University), weighed 120-150 g.

Levamlodipine benzenesulfonate and chlorthalidone were prepared into a stock suspension in 0.5% carboxymethyl cellulose (CMC), at a concentration of 10 times of that for intragastric administration, stored in dark in a freezer at 4°C for use, and diluted with 0.5% CMC before use.

### 1.2 Experimental method

Administration method: Each group of 8 animals was intragastrically administered at a dosage of 1 mL/kg following a clinical intragastric administration method for rats, and the control group was given equal volume of 0.5% CMC.

Blood pressure measurement method: The blood pressure of the rats was determined by using a MedLab®-MS60X model rat noninvasive tail artery blood pressure measurement system. Root of the tail of rats was placed under a heating lamp (100 W) for 5-10 min, and extended through a pressurization tail jacket after local vessels expanded fully. A fastening knob on a tail fastener was suitably screwed, such that center of a ventral surface of the rats closely contacted a pulse sensor of the noninvasive tail artery blood pressure measurement system, the pulse waveform of the system was observed at the same time, and the blood pressure was measured when the pulse waveform was stable. After the animals were quiet, the tail jacket was pressurized by charging air, it could be seen that the pulse wave gradually weakened to complete disappearance, and then the air was discharged till the pulse wave was completely restored. "Current data analysis" was clicked to obtain a result. The measurement was repeated 3 times, and the results were averaged.

Blood pressure measurement time: Each group of animals was determined for basic blood pressure in the morning, and intragastrically administered with the test medicine 30 min after the measurement of the basic blood pressure, and the effect of the medicine on blood pressure was observed respectively at 2, 6, 12, and 24 h after administration.

Experimental scheme: According to existing clinical experience, the effective dosage of levamlodipine benzenesulfonate was 3.465 mg/kg (containing levamlodipine 2.5 mg/kg), and the effective dosage of chlorthalidone was 12.5 mg/kg. Orthogonal design was used to determine the optimum proportion of levamlodipine and chlorthalidone. The dosage of levamlodipine benzenesulfonate was set at 1.386 mg/kg (containing levamlodipine 1.0 mg/kg), 3.465 mg/kg (containing levamlodipine 2.5 mg/kg), and 10.395 mg/kg (containing levamlodipine 7.5mg/kg) respectively; and the dosage of chlorthalidone was set at 5.0 mg/kg, 10.0 mg/kg, and 25.0 mg/kg respectively. The design is shown in Table 1.

**Table 1 Dosage formulation of levamlodipine and chlorthalidone**

| Dosage of Levamlodipine (L) (mg/kg) | Dosage of Chlorthalidone (C) (mg/kg) | | |
|---|---|---|---|
| | 5.0 | 10 | 25 |
| 1.0 | L1C5 | L1C10 | L1C25 |
| 2.5 | L2.5C5 | L2.5C10 | L2.SC25 |
| 7.5 | L7.5C5 | L7.5C10 | L7.SC25 |

Compound reparations of the above 9 formulations were respectively intragastrically administrated to 9 groups of rats, and the blood pressure was measured. The test results are shown in Table 2.

**Table 2 Changes of blood pressure of rats with time after administration of different formulations**

| Blood Pressure (mmHg) | Before Administration | 2h | 6h | 12h | 24h |
|---|---|---|---|---|---|
| L1C5 | 243±25 | 232±30 | 221±26 | 249±31 | 235±24 |
| L2.5C5 | 245±31 | 226±20 | 218±19 | 230±24 | 225±28 |
| L7.5C5 | 240±26 | 170±26 | 163±21 | 200±21 | 179±25 |
| L1C10 | 243±30 | 224±25 | 210±18 | 235±27 | 218±22 |
| L2.5C10 | 248±31 | 187±24 | 175±23 | 201±23 | 176±19 |
| L7.5C10 | 246±27 | 164±28 | 160±21 | 187±19 | 169±24 |
| L1C25 | 242±31 | 210±30 | 202±18 | 239±30 | 222±27 |
| L2.5C25 | 247±28 | 174±26 | 166±20 | 198±26 | 172±20 |
| L7.5C25 | 244±27 | 156±26 | 153±21 | 187±25 | 160±30 |

It can be known from Table 2 that 6 h after administration, the decrease of blood pressure in each group is greater than 20 mmHg, indicating that all pharmaceutical formulations are effective. 24 h after administration, the decrease in L1C5 group is lower than 20 mmHg, indicating that the combined efficacy cannot be maintained for 24 h. In addition, the decrease of blood pressure in L7.SC5, L7.SC10, L7.SC25, and L2.SC25 groups is greater than 60 mmHg, indicating that these combinations are unsuitable for common patients due to the excessive decrease. Therefore, the optimum formulation is levamlodipine 2.5-5.0 mg, and chlorthalidone 12.5-25.0 mg.

### Clinical Use Embodiment 1

The selected primary hypertension patients (with a systolic pressure ≥ 160 mmHg) were divided into 3 groups of 30 patients each, the levamlodipine group was given levamlodipine at 5.0 mg/d; the chlorthalidone group was given chlorthalidone at 25.0 mg/d; and the combined administration group was given levamlodipine at 2.5 mg/d and chlorthalidone at 12.5 mg/d. After 8-week treatment, the decease of the systolic pressure, the edema incidence, and the hypokalemia incidence were observed. The results are shown in Table 3 below.

**Table 3 Decrease of systolic pressure and incidence of side effects**

| Group | Decrease of Systolic Pressure (mmHg) | Edema Incidence (%) | Hypokalemia Incidence (%) |
|---|---|---|---|
| Levamlodipine Group | 22.7±7.8 | 10 | 0 |
| Chlorthalidone Group | 20.5±8.5 | 0 | 20.3 |
| Combined Administration Group | 29.6±10.4 | 0 | 6.7 |

It can be seen from Table 3 that, the decease in the combined administration group is the highest, and when the dosage of the combined administration group is a half of that of the levamlodipine group plus a half of that of the chlorthalidone group, the systolic pressure is obviously higher (P<0.05) than that of the levamlodipine group and the chlorthalidone, suggesting that the combined administration has a synergistic effect, and the hypertension depression effect is high. Furthermore, the edema incidence and the hypokalemia incidence in the combined administration group are respectively significantly lower than (P <0.05) those of the levamlodipine group and the chlorthalidone group, suggesting that the combined administration can decrease the incidence of adverse effects caused by the two medicines. Moreover, in the test, it is also found that the acting time in the combined administration group is obviously shorter than that of the levamlodipine group, and slightly longer than that of the chlorthalidone.

### Preparation Embodiments 1-6

Table 4 shows formulations for preparing 1000 levamlodipine and chlorthalidone compound tablets.

**Table 4 Formulations of levamlodipine and chlorthalidone compound tablets**

| Ingredient | Embodiment 1 | Embodiment 2 | Embodiment 3 |
|---|---|---|---|
| Levamlodipine Benzenesulfonate (g) | 1.74 (equivalent to levamlodipine 1.25) | 6.95 (equivalent to levamlodipine 5.00) | 6.94 (equivalent to levamlodipine 5.00) |
| Chlorthalidone (g) | 12.50 | 5.00 | 25.00 |
| Microcrystalline Cellulose (g) | 25.00 | 25.00 | 12.50 |
| Pregelatinized Starch (g) | 65.46 | 67.05 | 59.56 |
| Lactose (g) | 10.00 | 10.00 | 10.00 |
| Hydroxymethyl Starch Sodium (g) | 5.00 | 5.00 | 5.00 |
| Magnesium Stearate (g) | 1.00 | 1.00 | 1.00 |
| 95% Ethanol | Suitable amount | Suitable amount | Suitable amount |

Table 4 (continued) Formulations of levamlodipine and chlorthalidone compound tablets

| Ingredient | Embodiment4 | Embodiment5 | Embodiment6 |
|---|---|---|---|
| Levamlodipine Benzenesulfonate (g) | 3.47 (equivalent to levamlodipine 2.50) | 6.95 (equivalent to levamlodipine 5.00) | 3.47 (equivalent to levamlodipine 2.50) |
| Chlorthalidone (g) | 12.50 | 12.50 | 25.00 |
| Microcrystalline Cellulose (g) | 25.00 | 25.00 | 25.00 |
| Pregelatinized Starch (g) | 63.03 | 59.55 | 50.53 |
| Lactose (g) | 10.00 | 10.00 | 10.00 |
| Hydroxymethyl Starch Sodium (g) | 5.00 | 5.00 | 5.00 |
| Magnesium Stearate (g) | 1.00 | 1.00 | 1.00 |
| 95% Ethanol | Suitable amount | Suitable amount | Suitable amount |

Preparation process: Levamlodipine, chlorthalidone, microcrystalline cellulose, pregelatinized starch, lactose, and hydroxymethyl starch sodium were placed in a mortar, and ground and uniformly mixed, screened with a 20-mesh sieve, added into a suitable amount of 0.95% ethanol to obtain a soft material, screened with a 20-mesh sieve, granulated, and air dried at 40°C. The dried granules were finished with a 16-mesh sieve, added with magnesium stearate, uniformly mixed, and then tableted.

## Claims

1. A levamlodipine compound pharmaceutical composition, comprising levamlodipine or a pharmaceutically acceptable salt thereof and chlorthalidone.

2. The levamlodipine compound pharmaceutical composition according to claim 1, wherein the weight ratio of levamlodipine to chlorthalidone is 1:1-10.

3. The levamlodipine compound pharmaceutical composition according to claim 2, wherein the weight ratio of levamlodipine to chlorthalidone is 1: 5-7.5.

4. The levamlodipine compound pharmaceutical composition according to claim 1, wherein the pharmaceutically acceptable salt is one or more selected from acetate, benzenesulfonate, benzoate, citrate, fumarate, glucoheptonate, hydrochloride, lactate, maleate, malate, mesylate, nitrate, phosphate, succinate, phosphate, and tartrate.

5. The levamlodipine compound pharmaceutical composition according to claim 1, further comprising a pharmaceutically acceptable adjuvant.

6. The levamlodipine compound pharmaceutical composition according to claim 5, wherein the content of levamlodipine is 0.8-4.0 wt%; and the content of chlorthalidone is 4.0-40 wt%.

7. The levamlodipine compound pharmaceutical composition according to claim 6, wherein the content of levamlodipine is 2.0-4.0 wt%; and the content of chlorthalidone is 10-20 wt%.

8. The levamlodipine compound pharmaceutical composition according to claim 5, wherein the pharmaceutically acceptable adjuvant is one or more selected from microcrystalline cellulose, pregelatinized starch, lactose, hydroxymethyl starch sodium, and magnesium stearate.

9. The levamlodipine compound pharmaceutical composition according to claim 5, further comprising a pharmaceutically acceptable diluent, adhesive, disintegrant, lubricant, coloring agent and/or flavoring agent.

10. The levamlodipine compound pharmaceutical composition according to claim 5, wherein the pharmaceutical composition is a tablet or a capsule.
